# EUROPEAN PATENT APPLICATION

(11) **EP 1 266 667 A1**
(43) Date of publication of application: **18.12.2002**
(21) Application number: 00911360.6
(22) Date of filing: 24.03.2000
(51) Int. Cl.: A61L 27/40, A61L 27/56

(54) **ARTIFICIAL HOLLOW ORGAN**

(71) Applicant: Mori, Yuichi, Yokohama-shi, Kanagawa 236-0045 (JP)
(72) Inventor: Mori, Yuichi, Yokohama-shi, Kanagawa 236-0045 (JP)
(74) Representative: Wibbelmann, Jobst, Dr., Dipl.-Chem.
(86) International application number: JP0001834
(87) International publication number: WO01070292

(57) **Abstract**

An artificial lumen comprising a expansible member, and a tubular member of a porous material surrounding the expansible member, wherein easy insertion thereof into a living body lumen, and the characteristics to be satisfied by the artificial lumen after the insertion thereof into the living body lumen, are compatible with each other. The artificial lumen has a product of Q1 = (Sa × Sb × Sc) of 6 × 10⁴ or less, provided that Sa (mm) denotes the bending resistance of the porous material by the cantilever bending resistance testing method, Sb (mg/cm²) denotes the weight of the porous material, and Sc (ml•cm⁻²•min⁻¹) denotes the water-permeability of the porous material according to the ANSI/AAMI standard.

## Description

### Technical Field

The present invention relates to an artificial lumen (for example, vascular prosthesis or artificial blood vessel), particularly to an artificial lumen of a type such that it includes therein a stent, and it can temporarily or almost permanently be placed in the inside of a lumen (for example, blood vessel) of the body of an animal such as human so as to replace the functions of the animal lumen partially or entirely.

### Background Art

Many techniques have been developed in order to produce a stent or stent graft (or a member to be transplanted) which is to be temporarily or almost permanently placed in the inside of a lumen (for example, blood vessel) in a body.

For example, in a small area of the blood vessel, in the case of a stenosis of coronary artery and of peripheral artery of extremity, there is a method wherein a guide wire is inserted into a blood vessel, and then a stent is inserted along the guide wire to reach the stenosis portion so that the stenosis portion is dilated or expanded with a balloon, and the stent is placed in the stenosis portion. U.S. Patent No. 4,733,665 of Palmaz is a representative of such techniques.

As another representative techniques, there is a Gianturco-type stent technique wherein a stent having a zigzag structure is fabricated by using stainless steel elastic wires, and the stent is preliminarily inserted into a sheath (i.e., a catheter having a larger inside diameter). When the Gianturco-type stent is used, the stent is inserted into a lumen together with the sheath, and after the stent reaches an intended site in the lumen, the stent is discharged from the sheath so as to effect the self-expansion of the stent.

As described above, some devices to be used for the treatment for vascular wall wherein the device is inserted into a blood vessel by using a sheath so as to reach the intended lesion area of the blood vessel and to be dilated therein are described in the specifications of U.S. Patent Nos. 5,219,355; 5,211,658; 5,104,399; 5,078,726; 4,820,298; 4,787,899; 4,617,932, 4,562,596, 4,577,631; and 4,140,126; and European Patent Nos. 508473, 466518, and 461791; etc..

Another self-expanding type vascular stent which is capable of expanding spontaneously after the discharge thereof from a sheath is described in the specifications of U.S. Patent Nos. 5,147,370; 4,994,071; and 4,776,337; European Patent Nos. 575719; 556850; 540290; 536610; and 4813654; and German Patent No. DE 4219949; etc.

Further, in recent years, a technique wherein a shape-memory alloy is used, and a member comprising the shape-memory alloy is caused to have a temperature which is the same as the body temperature after the discharge of the shape-memory alloy member from a sheath so as to provide an originally intended shape of the shape-memory alloy member has been used even in the field of medical care. The use of such a shape-memory alloy is described in the specifications of U.S. Patent Nos. 4,665,906 and 4,505,767, etc.

As the clinical application of these stent, Parodi in Argentina has reported in 1991 for the purpose of treating aortic aneurysm in an aorta area, a technique wherein a stent capable of being expanded with a balloon manufactured by Palmaz is covered with a polyester cloth, and the resultant product is inserted into aorta as a transplanting member (Parodi JC, Palmaz JC, Baeone D, et al., Transfemoraointraluminal graft implantation for abdominal aortic aneurysms, Ann VascSurg 1991; 5:491-5). Further, Chuter had made a bifurcated-type stent and transplanting member for abdominalaortic aneurysm, and has reported their clinical application in 1994 (see, Chuter TAM, Donayre C, Wendt G, Bifurcated stent-grafts for endovascular repair of abdominalaortic aneurysm: preliminary case reports. Surg Endosc 1994; 8:800-2).

These technique have actually been applied in clinical uses to the treatment for aneurysm which has been considered difficult to be treated, and have showed good results. In these technique, there is adopted a method wherein a femoral artery or an iliac artery is incised or punctured, a catheter or a sheath is inserted through the incised or punctured site into the interior of a body, so as to reach a morbid site in the blood vessel.

Particularly, in the case of the treatment for abdominal aortic aneurysm or chest aortic aneurysm, a femoral artery or an iliac artery is incised, a catheter is inserted through the incised site into the blood vessel beyond the site of the aortic aneurysm, and thereafter the catheter is further inserted into the site which is nearer to the central site (nearer to the heart) than the site of the aortic aneurysm. Then, a stent graft is inserted thereinto through a sheath having a larger diameter.

Such a manipulation using a catheter or sheath can be conducted even in a case wherein laparotomy incision or thoracotomy incisionis is difficult, and therefore this manipulation has provided a new therapy which has not heretofore been considered. Based on such results, a new therapy called Endovascular Surgery has been established gradually.

However, even in the case of the new Endovascular Surgery therapy, there still remain some cases wherein the patients have not been cured completely by this therapy. One of the causes for the incompleteness resides in patients, and there are cases wherein the state of the patient is not suitable for this manipulation, e.g., a case wherein the artery wall of the patient is weak, and the artery wall is not so strong to bear the fixation of a stent to be placed therein.

The other one of the above causes is that there is still a room for improvement with respect to the device to be used for this therapy. The above problem encountered in the patient side is case-by-case. Therefore, in consideration of increase in the vascular disease which will possibly be caused along with the aging in the whole society, at the present stage, it is an urgent need to improve the device side (i.e., stent or stent graft).

The conventional stents or stent grafts have the following problems.
(1) The size of the stent graft is relatively large, and it inevitably provides a relatively large diameter even when the stent graft is folded. Therefore, when such a stent graft is intended to be inserted into the interior of a body, it is necessary to use a sheath having a relatively large diameter.
(2) The stent graft (as the entirety of the combination of a expanding member and a tubular member) is relatively rigid. Therefore, the stent graft cannot sufficiently follow the pathologic vascular wall of a patient which has been curved (or bent) and hardened.
(3) The adhesion property of the vascular wall with the stent graft is low, and therefore there is easily posed a phenomenon (Endoleak problem) such that blood leaks from the clearance between these vascular wall and stent graft.
(4) During or after the insertion of the stent graft into a blood vessel, the location of the stent graft can be deviated from the originally intended site for the stent graft in some cases.
(5) Based on the above "deviation", etc., the stent per se can block up the opening of a "collateral" (i.e., bifurcating part of a lumen) which is near to the site in the lumen where the stent has been placed.

An actual example of the incompleteness of the device, in the prior art, there is a type of a device such that when a stent and a transplanting member are carried by using a sheath into a site in a lumen where they are to be fixed, it is necessary to cause the stent and transplanting member to pass through the morbid site in the lumen, and to fix the stent, etc., at a site which is nearer to the center of the body. In this case, if the morbid site in the lumen is curved, it is necessary to bend the stent at the morbid site in accordance with the curvature of the morbid site in the lumen. At this time, if the sheath into which the stent and "folded transplanting member" have been inserted has a large diameter and is rigid, there can be involved a risk of rather damaging the morbid site in the lumen.

For example, the dissecting aneurysum tends to frequently occur in the vicinity of a site which is about 2 cm remote from the bifurcation site toward the peripheral side, at which a left subclavian artery (collateral) is branched from the aorta. Such a portion in the blood vessel is sharply curved, and therefore it is necessary to use a stent graft which can be flexibly bent, in order to fix the stent and transplanting member in this site by using the conventional technique. Further, in this case, it is necessary to take care that the left subclavian artery (collateral) should not be occuluded. In addition, it is necessary to fix the stent accurately and surely in a short section of the blood vessel.

In addition, the site at which the dissecting aneurysum is frequently caused is, as described above, the site having the sharpest curvature in the aorta, and therefore, it has been demanded to produce a stent and a transplanting member which can faithfully follow this curvature, and to insert and fix the stent and transplanting member safely. However, a stent or transplanting member which can completely satisfy such a demand has not been developed yet.

The above-mentioned circumstances concerning the stent or transplanting member are just the same as those concerning a stet-type artificial lumen which comprises a tubular member including therein a stent, and is a member which causes the tubular member to replace the functions of the lumen partially or entirely.

In order to solve such a difficult problem, the shape, feature, expanding method, material, etc., of the expansible stent have been improved, and new stent-type artificial lumens has been developed. Among these, the stent-type artificial lumen has been devised so that it can be inserted into a blood vessel at the time of the insertion thereof into the blood vessel, etc., even when the artificial lumen can be inserted from the blood vessel having a diameter as small as possible; it can be folded into a piece having a small diameter when the stent and the cloth are folded; and it can be caused to have a flexibility.

As a method of devising stent-type artificial lumen for such a purpose, some progresses have been found in the stent portion constituting the stent-type artificial lumen. However, in the improvement in the tubular member to be combined with the stent (usually, the tubular member comprises a cloth), no noticeable progress has been found.

As the improvement in the above "cloth portion", at present, the thickness of the conventional cloth is decreased. For example, there has been reported that a simple plain weave cloth is thinned to a thickness of about 0.2 mm and the raising (or piloerection) portion thereof is suppressed so as to provide a product having a flat and smooth surface structure.

However, when the cloth is thinned, the diameter of the multi-filament fibers constituting the cloth is inevitably decreased. Accordingly, in this case, there can be posed a serious problem that the strength of the cloth can also be decreased so that the water-permeability as the cloth per se is increased (therefore, the leakage of blood, etc., through the cloth is remarkably increased). In order to prevent a decrease in the strength of the cloth, it is inevitable that the number of the multi-filament fibers is reduced so as to provide a cloth having a simple knitted or woven structure and having a flat and smooth surface without having a raising state, in order to avoid a decrease in the diameter of the multi-filament fibers per se. However, it has heretofore not been pointed out such a "new" weak point which is caused by the above improvement in the cloth portion.

An object of the present invention is to provide a stent-type artificial lumen which has solved the above-mentioned problems encountered in the prior art.

Another object of the present invention is to provide a stent-type artificial lumen which has achieved the easy insertion thereof into a lumen of a living body (living organism), and the exhibition of good characteristics as an artificial lumen after the insertion thereof into the lumen of the living body, wherein the easy insertion and good characteristics are compatible with each other.

A further object of the present invention is to provide a stent-type artificial lumen wherein the tubular member thereof is thin but durable, and can easily maintain its original shape and form substantially; the artificial lumen can be folded into a small piece and can be inserted into a small-diameter sheath; and the artificial lumen can satisfy an affinity to the tissue of the living body, a thrombus-retaining property with respect to the thrombi residing in the wall of the artificial lumen, and an affinity to the cell of the living body.

### Disclosure of Invention

As a result of earnest study, the invention inventor has found that the use of a porous material (such as cloth) satisfying a combination of properties having a specified relationship is extremely effective for achieving the above-mentioned objects, instead of simply reducing the thickness of the tubular member of a stent-type artificial lumen as in prior art.

The artificial lumen according to the present invention is based on the above discovery, and comprises a expansible member, and a tubular member of a porous material surrounding the expansible member; the artificial lumen having a product of Q1 = (Sa × Sb × Sc) of 6 × 10⁴ or less, provided that Sa (mm) denotes the bending resistance of the porous material by the cantilever bending resistance testing method, Sb (mg/cm²) denotes the weight of the porous material, and Sc (ml•cm⁻²•min⁻¹) denotes the water-permeability of the porous material according to the ANSI/AAMI standard.

The present invention also provides an artificial lumen comprising a expansible member, and a tubular member of a porous material surrounding the expansible member; the artificial lumen having a product of combination Q2 = (Sa × Sb ÷ Sd) of 300 or less, provided that Sa (mm) denotes the bending resistance of the porous material by the cantilever bending resistance testing method, Sb (mg/cm²) denotes the weight of the porous material, and Sd (kg) denotes the fraying-out resistance of the porous material by the suture retention test according to the ANSI/AAMI standard.

The present invention further provides an artificial lumen comprising a expansible member, and a tubular member of a porous material surrounding the expansible member; wherein the cloth comprises fibers of 0.8 denier or less in an amount of 1% or more by the weight ratio.

The artificial lumen according to the present invention is based on the results of a discovery which have been obtained by earnest studies by the present inventor, concerning a problem which has never been investigated on artificial lumens.

More specifically, the present inventor has predicted problems of a conventional artificial lumen on the basis of knowledge related to the conventional artificial lumens, and as a result of these studies, the present inventor has found that in the case of a stent-type artificial lumen which is produced by merely decreasing the thickness of the cloth as a porous material so as to merely flatten and smoothen the cloth, a serious problem can be caused when the stent-type artificial lumen is actually inserted into a lumen in the living body such as blood vessel, and after a long period has elapsed from the insertion thereof.

More specifically, when the number of fibers constituting the tubular member is reduced, the cloth is thinned so as to provide a simple knitted or woven structure, there is cased a problem that the deformation or irregular shape of the weaving or knitting pattern (or network) is liable to occur. In order to thin the cloth, it is necessary to reduce the number of the fibers constituting the cloth, and to simplify the weaving or knitting pattern so as to reduce the three-dimensional thickness thereof. However, when the knitted structure or woven structure is simplified, the resultant cloth is liable to produce a disorder in the structure thereof due to weak stimuli, that is, the deformation of the cloth is liable to occur.

Apparently, the liablility of a stent-type artificial lumen to deform is not considered to be as an inconvenience. However, in such a case, the possibility of changing the form or shape of the weaving or knitting pattern of the cloth becomes higher without cutting the fiber, when certain stimuli are imparted to the artificial lumen. For example, there is supposed a case where a stent-type artificial lumen using a thin cloth which is liable to deform is inserted into a blood vessel through a small-diameter sheath, if the metal portion of the stent abuts on the cloth so that the metal portion cuts into the cloth (such a phenomenon is always liable to occur). When such a phenomenon occurs, although the metal portion dully cuts into the cloth, the metal portion will shift the weaving or knitting pattern structure of the thin cloth without cutting the fibers of the thin cloth, and the metal gradually pushes the weaving or knitting pattern of the cloth so as to widen the pattern, whereby the metal portion can be finally penetrated into the cloth.

Because the stent-type artificial lumen is inserted into a lumen of a living body, such a phenomenon can also occur while the artificial lumen is passing through a sheath. However, after the artificial lumen is passed through the sheath, the expansible portion of the artificial lumen is expanded in the blood vessel, and the metal portion returns to its original site (i.e., a state thereof before the insertion of the artificial lumen into the sheath). As a result, the state wherein the metal is penetrated into the cloth cannot be observed. However, there remain clearances and disorders in the running of the fibers caused by the widening of the pattern in the cloth by the metal. Actually, even if such a phenomenon has occurred, in the clinical practice, the stent-type artificial lumen have already been placed in the blood vessel, and the widened opening cannot be recognized by observing the stent-type artificial lumen with naked eye.

Accordingly, at present, a lot of manufacturers tend to produce artificial lumens which comprise a cloth having a thickness as thin as possible and have a flattened and smooth surface having a simple structure and having no raising so that they can pass through a sheath smoothly. However, it is considered that the above-mentioned thin cloth has a serious problem in the structure and strength thereof with respect to mechanical stimuli.

In other words, in the prior art, when the cloth is merely made thinner, the above-mentioned problem (penetration of the metal portion into the cloth) inevitably occurs. If the cloth is made flatter and thinner, it is seemed that the resultant artificial lumens have apparently been improved. However, even if the stent-type artificial lumen apparently provides good results at the time of the insertion of the artificial lumen, such an insertion is a dangerous action in a long-term point of view. Accordingly, it is quite possible that the improvement in the prior art cannot provide an essential improvement.

In addition, when the weaving or knitting pattern of a cloth is maintained to a dense level by further making the cloth thinner, the surface structure of the cloth is consequently made flat and smooth at the sacrifice of interspersed (or scattered) state and raising state of the fibers which function as an anchor for the adhesion of cells to the fibers. Consequently, the inner surface and the outer surface of the cloth can be made extremely flat. Based on the accumulated knowledge on the observations of healing processes after the implantation of artificial lumens, the present inventor has found out that the state provided by the above-mentioned thinned cloth can possibly provide a different state (that is, an undesirable state) from those provided by the former type of the artificial lumen evens in relation to cells, tissues, blood, thrombi , etc., after the insertion of the stent-type artificial lumen.

More specifically, in such a state wherein the metal portion is penetrated into the cloth and is protruded from the cloth, when the stent-type artificial lumen is fixed in the inner space of a blood vessel so that the artificial lumen is pushed outward to the wall of the blood vessel, the cells of the blood vessel wall which have received the resultant pressure fall into pressure necrose, or the alternation of generations of the cells and tissue is conducted at the site based on the recession thereof due to the pressure. As a result, a connective tissue will be formed in a manner so that the connective tissue receives the artificial lumen on the basis of weaker fixing force due to the pressure between the recessed connective tissue and the artificial lumen. In this case, if the outside surface of the cloth constituting the artificial lumen is a flat and smooth surface, cells cannot penetrate into the inside of the cloth, and therefore the cloth and the cells cannot show a so-called anchoring effect, i.e., an effect of firmly securing the cloth.

During a long term after the implantation of the stent-type artificial lumen, the stent-type artificial lumen always receives continuous stimuli due to the heartbeat, and also receives a force such as pressure from a surrounding tissue and contracting force from a scar tissue. Accordingly, it is pointed out that the stent-type artificial lumen causes a so-called graft migration, i.e., gradual shifting of the artificial lumen in the position thereof in the blood vessel. When the above anchoring effect is weak, it is difficult to prevent such a graft migration.

On the other hand, in the inner surface of the cloth, under a situation wherein the above-mentioned phenomenon is occurring on the outer surface of the cloth, if the inner surface is flat and smooth and has a low water-permeability, thrombus tissues adhering to the inside of the cloth cannot be rooted in the interstices or clearances between the fibers constituting the cloth, and it becomes difficult that the thrombus tissues continue existing stably on the cloth. As a result, there arises an unstable state wherein thrombus tissues are once attached to the cloth and are immediately detached therefrom, and such attachment and detachment are provided repetitively. Under these circumstances, even if cells are penetrated into the thrombus tissues and an organized tissue is formed fortunately, it will assume an unstable state due to the insufficient anchoring, and tissue pieces and thrombi are torn off from the cloth and are interspersed or distributed toward the peripheral system of a body, whereby a complication of embolism in the peripheral blood vessel is liable to occur. That is, an unstable state continues regarding the inside of the cloth, in the same manner as in the outside thereof.

As described above, the improvement in the cloth portion of an artificial lumen has seriously been delayed, and in view of most of the products, it is the present situation that the artificial lumens which should originally be used for open surgery (i.e., a surgical operation to be conducted while opening the affected portion) are still misappropriated also in Endovascular Surgery inevitably. Accordingly, in the present clinical practice, the devices for the open surgery are used, and therefore they are caught in a dilemma such that it is essentially difficult to make the whole devices thinner and to insert the stent-type artificial lumen by using a small-diameter sheath.

### Brief Description of Drawings

Fig. 1 is a schematic perspective view showing an embodiment of the stent-type artificial lumen according to the present invention, which is constituted by super ultra-fine fibers and a Z-type metal stent.
Fig. 2 is a schematic sectional view showing an example of the cloth constituting the artificial lumen according to the present invention, which has super ultra-fine fibers 4a, 4b, 7 and 8.
Fig. 3 is a schematic sectional view showing the cloth constituting the artificial lumen which have been used as Reference product A in Examples appearing hereinafter.
Fig. 4 is a schematic sectional view showing the cloth constituting the artificial lumen which have been used as Reference product B in Examples appearing hereinafter.
Fig. 5 is a schematic sectional view showing the cloth constituting the artificial lumen which have been used as reference product C in Examples appearing hereinafter.
Fig. 6 is a schematic perspective view showing a cantilever-type testing machine to be used for textile bending resistance test in the present invention.

In these figures, each reference numerals have the following meanings:
1: artificial lumen,
2: expansible member (stent),
3: tubular member,
4a: raised super ultra-fine fibers (in a state wherein the super ultra-fine fibers are raised so as to protrude a cutting edge thereof from the cloth),
4b: raised super ultra-fine fibers (in a state wherein the super ultra-fine fibers are raised so as to form a loop from the cloth),
5: fibers having an ordinary diameter (woof of plain weave structure),
6: fibers having an ordinary diameter (warp of plain weave structure),
7: super ultra-fine fibers (inside longitudinal cross section of cloth),
8: super ultra-fine fibers (inside lateral cross section of cloth),
9: fibers having an ordinary diameter of the Reference product A (woof of plain weave structure),
10: fibers having an ordinary diameter of the Reference product A (warp of plain weave structure),
11: fibers having an ordinary diameter of the Reference product B (woof of plain weave structure),
12: fibers having an ordinary diameter of the Reference product B (warp of plain weave structure),
13: fibers having an ordinary diameter of the Reference product C (woof of plain weave structure),
14: fibers having an ordinary diameter of the Reference product C (woof of plain weave structure, which have been raised so as to form a loop), and
15: fibers having an ordinary diameter of the Reference product C (warp of plain weave structure),

### Best Mode for Carrying Out the Invention

Hereinbelow, the present invention will be described in detail with reference to the accompanying drawings as desired. In the following description, "%" and "part(s)" representing a quantitative proportion or ratio are those based on mass (or weight), unless otherwise noted specifically.

### (Artificial lumen)

The artificial lumen according to the present invention comprises a expansible member, and a tubular member of a porous material surrounding the expansible member.

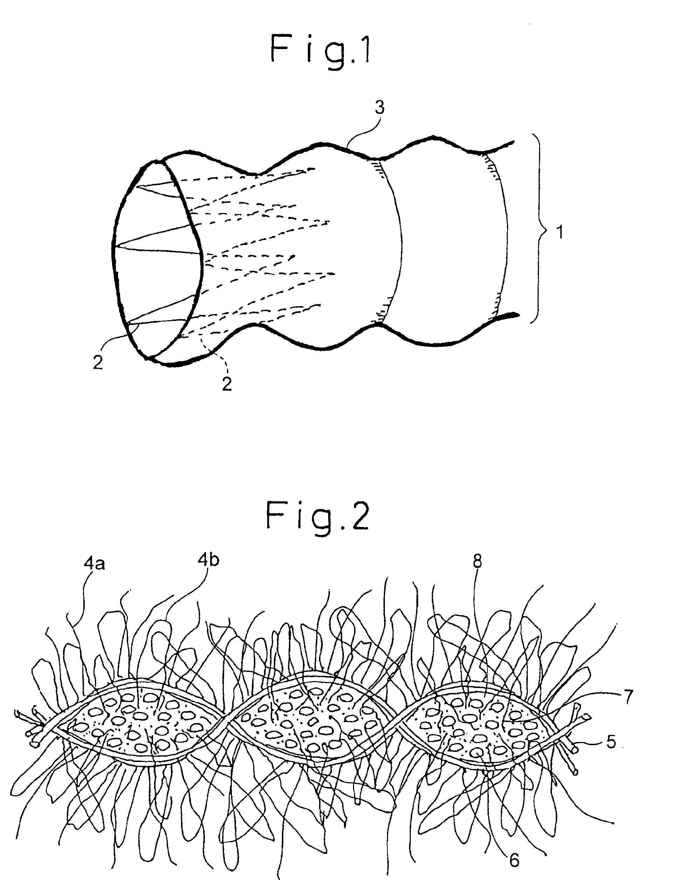

Fig. 1 is a schematic perspective view showing a basic embodiment of the artificial lumen according to the present invention. Referring to Fig. 1, the artificial lumen in such an embodiment comprises a expansible member 2 (only a portion thereof is shown), and a tubular member 3 of a porous material surrounding the expansible member 2 .

### (Expansible member)

As the expansible member 2, it is possible to use a member appropriately selected from those known in the art (for example, stent-type member), which is expansible in a lumen.

The properties, material, diameter, etc., of the linear member constituting the expansible member 2 are not particularly limited, as long as the linear member can be formed into the above expansible member 2, and the material can be appropriately selected from those known in the art. The material constituting the expansible member generally comprises a metal (this term is used so as to include an alloy), but the materials usable in the present invention are not limited to metals.

A part or the entirety of the linear member may preferably comprise a material having a shape-memory function (for example, shape-memory alloy) or a super elastic material (for example, super elastic alloy), since such a material can facilitate the function of the stent such that when the stent is inserted into a lumen, the stent assumes a desired shape at a predetermined temperature.

Further, as desired, the above linear member can take any one of known shapes/structures such as mono filament, multi-filament, and tape (or any of combinations of two of more species of these structures). When such a measure is adopted, it is possible to select and regulate the flexibility, restoring force, etc., of the linear member, and it is easy to make a expansible member having a curved or bent surface which is adapted to a curved surface of the soft lumen (for example, blood vessel) inner wall.

### (Dimension)

The dimension or size of the expansible member 2 is not particularly limited, as long as it can be inserted into a lumen.

### (Production process)

The process for producing the expansible member 2 according to the present invention is not particularly limited, as long as the process can impart the above-mentioned predetermined shape to the expansible member, and the process can use one (as desired, as a combination of two or more kinds) which is appropriately selected from known materials (such as metals) and known manufacturing method.

The method for expanding the expansible member may be any of types such as self-expanding type, and one which is expanded by another device such as balloon.

### (Tubular member)

In view of the compatibility of easy insertion into a lumen in a living body, with the property of the artificial lumen after the insertion thereof into a lumen (by the suppression of the penetration/deformation of the tubular member by the expansible member), the porous material constituting the tubular member 3 may preferably show a value of the product Q1 = (Sa × Sb × Sc) of 6 × 10⁴ or less, more preferably 5 × 10⁴ or less (particularly 4 × 10⁴ or less), provided that Sa (mm) denotes a bending resistance according to the 45 degree-cantilever bending resistance testing method as described in JIS (L 1096-1990, general textile testing methods), Sb (mg/cm²) denotes the weight of the above-mentioned porous material, and Sc (ml•cm⁻²•min⁻¹) denotes the water-permeability of the porous material according to ANSI/AAMI standard.

In view of the compatibility of easy insertion into a lumen in a living body, with the property of the artificial lumen after the insertion thereof into a lumen, in the present invention, the porous material constituting the tubular member 3 may preferably show a value of the combination of Q2 = (Sa × Sb ÷ Sd) may be 300 or less, provided that Sa (mm) denotes the above-mentioned bending resistance, Sb (mg/cm²) denotes the weight of the above-mentioned porous material, and Sd (kg) denotes the fraying-out resistance of the porous material. Further, the value of Q2 may preferably be 250 or less (particularly 200 or less).

Each of these physical properties, bending resistance Sa, weight Sb, water-permeability Sc and fraying-out resistance Sd of the porous material may be measured in the following mannero.

### <Method of measuring bending resistance Sa>

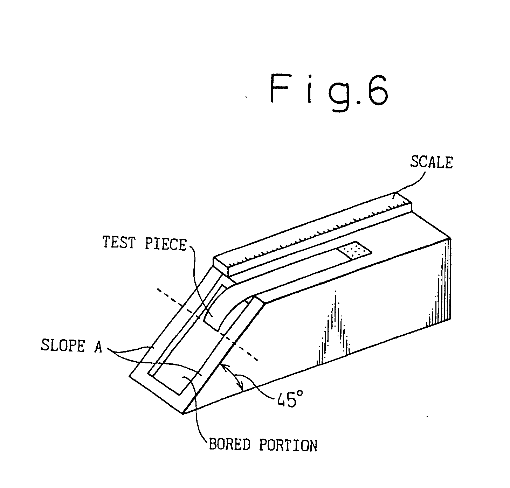

The bending resistance is measured by the "45° - cantilever method". Test pieces each having a size of about 2cm width × about 15cm length are taken out from the sample of which bending resistance Sa is to be measured (porous material such as cloth) with respect to the wales direction (five or more sample pieces) and the course direction (five or more sample pieces) of the sample, respectively. Then, each of the sample pieces is placed on a horizontal mount having a smooth surface with a slope of 45 degrees at one end of the surface so that the shorter side of the sample piece is aligned with the scale base line of the mount, as shown in the schematic perspective view of Fig. 6. The test piece is slowly moved to slide on the surface toward the slope by using a suitable measure, and when the middle point of one end of the test piece comes into contact with the slope A, the position of the other end of the test piece is read by means of the scale of the mount. The bending resistance is indicated by the length (mm) of the test piece during which the test piece has moved, and the value of the bending resistance is expressed by the average value with respect to the front and back surfaces of the test piece (so as to provide a value to the integer units' place).

### <Method of measuring weight Sb of porous material>

The weight of a porous material (such as cloth) of 2cm width × 15cm length which has been dried for about 24 hours or more under the conditions of about 25°C, and about 10% RH, is measured by means of an electronic weighing instrument (e.g., mfd. by Sartorius Co., trade name: BP 210S electronic balance; or mfd. by Chyo Balance Co., trade name: JL-200) which can provide a measured value to 0.1 mg place, and 1/30 of the measured value of the weight is treated as the weight per 1 cm².

### <Method of measuring water-permeability Sc>

A water stream having a water pressure equivalent to 120 mm Hg (16.0 kPa) pressure is applied to the surface of a sample of 0.5 - 1.0 cm² to be measured, and the quantity of water passing through the sample surface is measured for 60 seconds, and the measured quantity of water is converted into a value per a porous material of 1.00 cm².

### <Method of measuring fraying-out resistance Sd>

The fraying-out resistance Sd (kg) is measured by the suture retention test according to ANSI/AAMI (American National Standards Institute/Association for the Advancement of Medical Instrumentation) standard.

### (Material of tubular member)

The material and form of the tubular member 3 is not particularly limited. In view of easiness wherein the tubular member 3 is bent in accordance with the curved or bent lumen such as blood vessel, the tubular member 3 may preferably be constituted by a material/form having a flexibility. For example, when the tubular member 3 comprises a cloth or film, it is preferred to impart a flexing property to these materials by forming the material into a bellows-type configuration. For example, when the tubular member 3 comprises a material (for example, e-PTFE) having a fibrous property, it is preferred to impart flexing property thereto by appropriately selecting the fibril length. Herein, the "e-PTFE" refers to expanded polytetrafluoroethylene, that is, a material which has been obtained by suddenly stretching a tube of polytetrafluoroethylene (Teflon) so as to form innumerable cracks, and to provide a porous state thereto, and to provide a flexing property to the material. As the e-PTFE, it is generally possible to use, e.g., those sold under the trade name of "Goretex" (U.S.A., Gore Company).

### (Ultra-fine fibers)

In the present invention, the material, diameter, thickness, etc., of various kinds of the porous materials or fibers which can constitute the tubular member 3 are not particularly limited, as long as at least one of the relationships of Q1 ≦ 6 × 10⁴ or Q2 ≦ 300. The present inventor has earnestly investigated the compatibility of porous materials or fibers with the easy insertion thereof into a living body lumen and the property of the artificial lumen after the insertion thereof into a lumen. As a result, the present inventor has found that the specific relationship of Q1 or Q2 can easily be realized by using a specific amount of specific ultra-fine fibers.

More specifically, in an embodiment of the present invention wherein such ultra-fine fibers are used, the tubular member 3 may preferably comprise (fine) fibers of 0.8 denier or less in a weight ratio of 1% or more. The content of these fibers of 0.8 denier or less may more preferably be 2% or more, particularly 3% or more.

In view of easy provision of the raising structure, the tubular member 3 may preferably comprise extremely fine fibers of 0.5 denier or less in an amount of 1% or more, more preferably 3% or more, particularly 5% or more.

In such an embodiment of the present invention, instead of merely thinning the porous material as in the prior art, a predetermined amount of predetermined fine fibers are caused to be mixed in other fibers constituting the cloth, so as to design the structure of the cloth which can induce a good anchoring state of cells and thrombi.

The diameter of the fibers (denier) constituting the tubular member 3 and the weight ratio in the fibers can be confirmed, e.g., by the following method.

### <Method of confirming fiber diameter>

A sample cloth is embedded in epoxy resin, methyl methacrylate resin or hydrophilic PHEMA (polyhydroxyethyl methacrylate), and the resultant product is sliced with a glass knife into thin sample pieces having a thickness of 1 - 2 µm, and the resultant thin sample pieces are observed with an optical microscope (maginification: about 40-200 times). Among the observed fiber diameters, minimum ten diameter values of cross section of the fibers are selected, and these ten values are averaged.

### <Method of confirming fiber weight ratio>

Thinly sliced sample pieces are observed with an optical microscope in the same manner as described above. The number of ultra-fine fibers (for example, 0.8 denier or less) and the number of ordinary fibers other than the ultra-fine fibers are respectively measured, with respect to randomly selected ten areas having a dimension of 10 mm².

Then, the average diameters of the respective fibers are calculated, and the total amounts of the ultra-fine fibers and other fibers are calculated on the basis of the thus obtained average diameters, to thereby calculate the weight ratio therebetween. In this case, the calculation is conducted under the assumption that the mass per unit is the same, if the kind (raw material) of the ultra-fine fibers is the same as that of the fibers having an ordinary diameter.

As described above, in the present invention, the cloth constituting the tubular member 3 can be realized, e.g., by causing the fine fibers of 0.8 denier or less to be mixed in the cloth in an amount of 1% or more. The ordinary fibers (not ultra-fine fibers) have a diameter of 1.2 to 2.0 denier, and the diameter of the cross section of the fibers was found to be 16 microns to 20 microns.

In the present invention, e.g., in an embodiment wherein fine fibers of 0.8 denier or less are used, they provide a fiber diameter of 5 microns or less (fine fibers of 0.5 denier or less provide a fiber diameter of 4 microns or less). However, when the fine fibers are present in an amount of as small as 1 % in terms of the weight ratio, the number of the fine fibers constitutes 3 % or more of the total number of fibers. Further, when the ultra-fine fibers of 0.1 denier or less are present in an amount of 5 % in terms of the weight ratio, the number of the ultra-fine fibers is much larger than the number of the other fibers, that is, in this case, the tubular member 3 comprises 50% or more of ultra-fine fibers in terms of number of fibers.

### (Material of cloth)

In view of non-carcinogenic property, non-reactivity with foreign substance, non-degrading property, non-deteriorating property, non-swelling property, non-deforming property, etc., the tubular member 3 (in an embodiment wherein the tubular member 3 comprises a cloth) may preferably comprises fibers of a polymer of polyester, polyamide, or polyolefin.

### (Form of cloth)

In the present invention, the diameter, material, weaving or knitting method therefor, etc., of fibers constituting the cloth are not particularly limited, as long as the cloth satisfies predetermined physical properties. More specifically, in the present invention, it is possible to use, e.g., any of knitted webs, woven fabrics, or non-woven fabrics. In view of less liability in causing fraying-out in the cut edge of the cloth, non-woven fabric may preferably be used.

Among the non-woven fabrics, it is particularly preferred to use non-woven fabric comprising fine fibers which have been entangled or intermingled randomly or disorderly by use of high-pressure water jet, etc. In the cloth of such an embodiment, the cut edge thereof is particularly less liable to fray out, so that the prevention of cloth deformation becomes easier in view of the cloth structure. The above-mentioned entanglement of fibers (particularly, extremely complicated entanglement) becomes easier when the tubular member 3 includes the extremely fine fibers of 0.8 denier or less in an amount of 1% or more.

Some artificial lumens using super ultra-fine fibers in the prior art are described in Japanese Patent No. 906347 (Examined Patent Publication (KOKOKU) Hei. 4-59899), U.S. Patent No. 4,695,280 and European Patent No. 128,741. Based on these techniques, it becomes possible to impart a flexibility, an intima healing-accelerating effect, etc, to an artificial lumen by using fine fibers. Accordingly, the above Japanese Patent No. 906347 (Examined Patent Publication (KOKOKU) Hei. 4-59899), U.S. Patent No. 4,695,280 and European Patent No. 128,741 use super ultra-fine fibers. However, the purpose of the use, and the resultant effect obtained by such use, are utterly different from those in the present invention. That is, the limit to the thickness of the artificial lumen, etc., are not considered.

More specifically, the stent-type artificial lumen according to the present invention provides a novel effect to enhance the anchoring effect of the surrounding tissue and thrombi by causing the thickness of the cloth constituting the artificial lumen to made thinner, while maintaining the mechanical strength of the cloth.

### (Constant load gauge determination of wall thickness)

It is preferred that the cloth constituting the tubular member 3 provides a thickness as small as possible under a pressure, so that the tubular member is liable to be inserted into a sheath. For achieving such a purpose, the tubular member 3 provides a thickness as small as possible when it is pressed. More specifically, the thickness may preferably be suppressed to C = 0.2 mm or less (more preferably 0.15 mm or less) when it is measured by the constant load gauge determination of wall thickness according to ISO 5084.

On the other hand, it is preferred that the artificial lumen according to the present invention is caused to exhibit an anchoring effect after it is placed in a blood vessel, etc., so that the artificial lumen provides a room or field for the penetration of cells without liberating thrombi. In order to satisfy the structure for providing such a state, it is preferred to maintain a state wherein the cloth shows a thickness of diameter of M = 0.4 mm or more (more preferably, 0.5 mm or more) by the microscopic determination of wall thickness according to ISO 5081.

The ratio (M/C) of the above-mentioned two kinds of wall thicknesses may preferably be about 1.1 - 6, more preferably about 2 - 5 (particularly about 2 - 4 ).

In order to easily realize the two kinds of demands which can conflict with each other, the cloth constituting the tubular member may preferably comprise super ultra-fine fibers (of 0.8 denier or less) in an amount of 1 mass % or more. The super ultra-fine fibers can easily be pressed, but on the other hand, the super ultra-fine fibers have a property that the clearances or interstices between the fibers are liable to expand when the pressure is removed. This fact is clearly understood from a phenomenon commonly observed by everyone that a cloth made of thick fibers is generally less liable to be folded, and wrinkles are liable to remain in the cloth when it is forcedly folded; but a cloth made of fine fibers is liable to be folded, and wrinkles are less liable to remain in the cloth even after the folding. As a result, the ultra-fine fibers of which fiber clearances have been widened can easily retain cells and thrombi therein, so as to cause them to show an anchoring effect. In order to enhance this effect, it is advantageous to cause the super ultra-fine fibers to assume a raising state as described hereinafter. Even when the super ultra-fine fibers assume a high-raising state, it can be flattened under a pressure. In addition, such a cloth does not increase the total thickness thereof substantially at the time of being pressed.

In order to satisfy the above-mentioned conditions possibly conflicting with each other, it is the most preferred to use super ultra-fine fibers (of 0.8 denier or less). Conventional polyester fibers, polyolefin fibers, and polyamide fibers have a diameter of about 1.2 to 2.0 denier. In the case of such conventional thick fibers, the thinning of the fibers under pressure is rather limited. Even if these fibers are formed in a raising state, the recovery of the cloth thickness after the removal of pressure is limited, and even when the fibers in the raising state are flattened, a certain thickness still remains. However, when a cloth is made from super ultra-fine fibers, it is possible that the thickness of the cloth is suppressed under pressure, and the thickness may recover under removal of a pressure. The present inventor has investigated the diameter of ultra-fine fibers which does not substantially affect an increase in the cloth thickness. As a result, it has been found that both of the above-mentioned conditions possibly conflicting with each other may easily be satisfied by causing fibers of 0.8 denier or less, more preferably 0.5 denier or less (particularly 0.4 denier or less) to be mixed in an amount of at least 1% or more, more preferably 3% or more (particularly 5% or more) by the weight ratio, while such an amount may possibly be affected by the diameter of the super ultra-fine fibers, and the total number of fibers.

The super ultra-fine fibers have a much smaller diameter as compared with that of ordinary fibers. For example, ordinary polyester fibers have a diameter of about 1.2 to 2.0 denier. The diameter of the cross section of such fibers is 16 to 20 microns. On the other hand, the diameter of the cross section of the ultra-fine fibers is about 3 microns in the case of ultra-fine polyester fibers of 0.2 denier.

In a general point of view, there is a room for consideration such that when the diameter of the cross section of the super ultra-fine fibers is decreased, the tensile strength thereof becomes weak in proportion to the diameter decrease.

However, in view of the characteristic of fibers, as the diameter of the fibers is intended to be decreased to a smaller value, the material of the fibers are stretched more strongly in the production process for the fibers. In such a case, the respective polymer molecules in the fibers begin to be oriented regularly along the major axis direction of the fibers. In the present invention, such a special phenomenon can be utilized. It is known that the strength of fibers is remarkably enhanced in such a case.

Accordingly, in the present invention, even when the super ultra-fine fibers are used, it is substantially impossible that the super ultra-fine fibers are seriously inferior to the ordinary fibers in the strength thereof in proportion to the decrease in the diameter thereof, and the super ultra-fine fibers do not substitute the ordinary fibers. Accordingly, in the present invention, the super ultra-fine fibers have a larger flexibility than the ordinary fibers, but the strength of the ultra-fine fibers is not decreased, as the diameter of the super ultra-fine fibers becomes smaller. The above embodiment of the present invention using the ultra-fine fibers effectively utilizes the characteristic of fibers such that even when the diameter of the fibers is reduced, the strength of the fibers can be increased but the flexibility of the fibers is not decreased.

### (Folding property)

The tubular member 3 to be used in the present invention may preferably comprise a cloth having a characteristic which is equal to or more than the folding property of a cloth such that when the cloth is formed into a tubular member having an inside diameter of 20 mm, it has at least a folding property wherein the 20 mm-diameter tubular cloth can be formed into a small folded piece which can be inserted into at least a catheter of 12F (French; 1 French = 1/3 mm).

### (Strength of cloth)

At a least a portion of the cloth constituting the tubular member 3 may preferably have a the strength of 6g/d or more (with respect to the details of the strength, Toshihiko Ota et al., "High-Strength/High Elasticity Modulus Fibers", edited by The Society of Polymer Science, Japan, Kyoritsu Shuppan, 1988 may be referred to). Examples of such fibers may include, e.g., aromatic-type fibers of aramide available from of DUPONT Company, U.S.A. (trade name: Kevler), etc.

### (Raising)

In order to easily realize the easy insertion of the artificial lumen into the lumen in a living body, in compatibility with the characteristic as the artificial lumen after the insertion thereof into a lumen, in the present invention, the cloth constituting the tubular member may preferably be subjected to a "raising" treatment. The method of conducting the "raising" treatment is not particularly limited. It is possible to use one or a combination of two or more species which can appropriately be selected from known raising methods.

As describe hereinabove, during a long term, the stent-type artificial lumen always receives continuous stimuli due to the heartbeat, and also receives a force such as pressure from a surrounding tissue and contracting force from a scar tissue. Accordingly, the stent-type artificial lumen can cause a so-called graft migration, i.e., gradual shifting in the position of the artificial lumen. When the above-mentioned anchoring effect is weak, it is difficult to prevent the graft migration.

The phenomenon of the anchoring weakening frequently occurs in the case of a cloth which has a low water-permeability and has no raising state. In the case of a general artificial lumen (including no expansible member), it is not necessary to thin the wall of the lumen, unlike in the case of the stent-type artificial lumen. Accordingly, in the case of a general artificial lumen, cells are caused to penetrate into the weaving pattern of the lumen wall by causing the cloth into a sufficiently raising state, or enhancing the water-permeability of the cloth, and therefore it is expected that the cloth is integrated or unified with the connective tissue produced from the cells, whereby the anchoring of the cloth becomes good. However, in the case of the conventional stent-type artificial lumen, as described above, the outer surface of the cloth constituting the stent-type artificial lumen inevitably becomes flat and smooth, and therefore there arises a problem that the above-mentioned anchoring is weakened.

The state of low water-permeability without raising can provide an inconvenience also in the inner surface of the cloth. More specifically, when the implantation is conducted successfully, and the cloth is inserted into the inside of a blood vessel, it is generally expected that thrombi adhere to the inner surface of the cloth portion, and are organized to form a membrane comprising a stable tissue of the living body itself. It is extremely rare that the inner surface of the membrane is covered with endothelial cells, and therefore, in most cases, it is usual that the inner surface of the membrane is still constituted by thrombus tissues, or the inner surface is not accompanied with the covering of the endothelial cells, but the connective tissue only adheres to the inner surface.

In the present invention, instead of merely thinning the cloth in the prior art, e.g., extremely fine fibers of 0.8 denier or less are caused to be mixed in the fibers constituting the cloth, so as to design the structure of the cloth which can realize predetermined properties as the cloth and can induce a good anchoring state of cells and thrombi.

Further, in the present invention, as another effect of random entanglement of fine fibers due to the raising, the deformation of the cloth is prevented so as to prevent a complication such as endoleak which can occur after the implantation of the artificial lumen.

### (Suture retention test)

Further, the artificial lumen according to the present invention may preferably have a suture retention of 0.5 kg or more by the suture retention test according to ANSI/AAMI standard, which is equal to or more than the fraying-out resistance of the porous material artificial lumen to be used for ordinary open surgery. As described above, this condition cannot be satisfied by simple structures in the artificial lumen made of a thin cloth.

In the present invention, such a condition may easily be satisfied by utilizing the random and complex entanglement of super ultra-fine fibers in the inside and the surface portion of the cloth constituting the artificial lumen. As described above, the fine fibers are durable in view of the strength thereof, although they have a small diameter. The present inventor has found a phenomenon even in a thin cloth that when the fine fibers are entangled randomly and complicatedly, even when a force is applied to the entangled fibers in any direction, these fibers become entangled tightly so that the entanglement between the fibers become more tightly as the stimuli to be applied thereto become stronger, and as a result, an innumerable knots are produced, and therefore the fraying-out is much less liable to occur.

In addition, it has also been found that the entanglement of the fibers becomes more random and more complicated, as the diameter of the fine fibers becomes smaller. As a result of the present inventor's investigation on the diameters of the fibers, it has been found that when the tubular member 3 comprises fibers of 0.8 denier or less, more preferably 0.5 denier or less (particularly 0.4 denier or less) in an amount of 1% or more, such complex and random entanglement of the fibers is easily obtained so as to provide a fraying-out resistance of 0.5 kg or more by the suture retention test according to ANSI/AAMI standard.

It is not easy to numerically represent the degree of the easiness in the deformation of such a cloth, but it is possible to use another method as an index to show the degree of the easiness in the intersperseing of the cloth. At the cut edge of the cloth, the degree of the easiness in the deformation can be expressed by the fraying-out resistance. Accordingly, it is possible that the artificial lumen is cut off so that the threads are obliquely cut, and the fraying-out resistance at the site which is 2 mm remote from the resultant cut edge is alternatively used as the degree of easiness in the deformation of the cloth.

That is, if the fraying-out resistance at the cut edge is higher, it is estimated the cloth is less liable to deform when it is in a flattened state. The present inventor has found that when the fraying-out resistance at the cut edge is measured from such a viewpoint, the artificial lumen for general open surgery shows a resistance value of about 0.5 kg, but a thin cloth (thickness of about 0.4 mm) having a conventional plain weave structure shows a resistance of 0.3 kg or less.

When the strength of a thin cloth comprising entangled super ultra-fine fibers is measured in this way, it has been found that the inclusion of fibers of 0.8 denier or less, more preferably 0.5 denier or less (particularly 0.4 denier or less) in an amount of 1% or more by weight ratio is necessary. As a result of the present inventor's investigation, it has been found that such a state can easily be obtained by effectively utilizing the method of entangling fibers by use of water jet as described in Japanese Patent No. 906347 (Examined Patent Publication (KOKOKU) Hei. 4-59899), U.S. Patent No. 4,695,280 and European Patent No. 128,741. Further, As a result of the present inventor's investigation, it has also been found that the cloth which has been obtained in this manner may easily provide a pressure resistance of 20 kg or more by the burst test according to ANSI/AAMI standard.

In order to produce a cloth wherein super ultra-fine fibers are entangled in such a state, it is convenient that the fibers are polyester, polyamide, or polyolefin, but the fibers are not necessarily limited to these materials. It is possible to practice the present technique for obtaining the entanglement of super ultra-fine fibers, as long as the material for the fibers can be implanted into a living body.

The entanglement of super ultra-fine fibers may be produced, e.g., by two kinds of methods.

One of these methods is a method wherein super ultra-fine fibers are interwoven or interknitted in a tubular member of a cloth from the beginning of the production process, as described in Japanese Patent No. 906347 (Examined Patent Publication (KOKOKU) Hei. 4-59899), U.S. Patent No. 4,695,280 and European Patent No. 128,741, and after the formation of the cloth, the cloth is subjected to a raising treatment so that the fibers are interspersed by the raising technique. When such a treatment is conducted, it is possible to obtain a state wherein innumerable super ultra-fine fibers have been raised from the surface of the cloth. Further, in order to cause these fibers to be mutually entangled randomly and strongly, the resultant cloth is subjected to water jet processing using high-pressure water, whereby the flexible super ultra-fine fibers are liable to raise. As a result, it can be observed that in the both sides of the resultant cloth, the raised super ultra-fine fibers are formed into loop-like shapes, or each of these fibers is cut so as to provide the resultant standing-up cut edges; and these tow states are co-present.

The other method of the above two methods is a method wherein a cloth having a simple woven or knitted structure is produced at first, and then non-woven fabric made of super ultra-fine fibers is superposed on the cloth of the simple woven or knitted structure, and the thus obtained superposition is subjected to a water jet treatment using high-pressure water. When such an operation is conducted, the non-woven fabric of super ultra-fine fibers is entangled into the respective fiber clearances of the base material, and integrated or unified with the base material, so as to finally provide a raising state wherein the super ultra-fine fibers are stood up from the base material. Whether either of the above two methods is used, a state wherein the super ultra-fine fibers are raised from both sides of the cloth is provided.

The thus obtained cloth is formed into a tubular product, but the tubular product is liable to kink when it is curved or bent. Accordingly, it is preferred to subject the tubular product to a crimping treatment for the purpose of preventing the kink. In a stent-type artificial lumen which is selectively used for a straight portion of the lumen in a living body, the crimp structure is less useful, as compared with that in the case of an artificial lumen which is selectively used for a curved or bent portion of the lumen in a living body.

### (Water-permeability)

The tubular member 3 may preferably have a water-permeability (or water passing property) of 1000 ml•cm⁻²•min⁻¹ or less, more preferably 500 ml•cm⁻²•min⁻¹ or less, particularly 200 ml•cm⁻²•min⁻¹ or less according to ANSI/AAMI. However, in the present invention, the super ultra-fine fibers are entangled with the cloth, it is considered that the leak of blood from the fiber clearances of the weaving or knitting pattern of the actual cloth is much less than that in the case of ordinary porous material artificial lumen having the same water-permeability.

In the present invention, in a case where the cloth has a water-permeability of 500 ml•cm⁻²•min⁻¹ or less per 1 square centimeter, when a water pressure corresponding to a pressure of 120 mmHg is applied thereto, it is preferred to use a tubular member 3 having a cloth thickness which can be inserted into a thin catheter of 12F or less when the cloth is formed into a tubular member having an inside diameter of 20 mm.

The super ultra-fine fibers which have been entangled in this manner have a characteristic that the super ultra-fine fibers are completely interspersed or scattered by the water jet processing, and therefore the extremely fine fibers are present in the form of spider's web in the clearances or interstices between the fibers, so that extremely fine fibers are everywhere entangled. Accordingly, the super ultra-fine fibers have a characteristic that thrombi are liable to adhere to the fibers even if they transmit water, and therefore they selectively exhibit thrombus-retaining ability and the leak of blood is markedly decreased. Unexamined Patent Publication (KOKAI) Sho. 63-115555 teaches that the use of super ultra-fine fibers can provide such a characteristic.

### (Dimension or size)

When the artificial lumen according to the present invention is actually used for a human living body, the size thereof usually relates to a range of not smaller than 3 mm and not larger than 40 mm in terms of the inside diameter thereof. However, the artificial lumen to be used is not necessarily in this range depending on its usage.

### (Fixing of tubular member with expansible member)

As desired, the above-mentioned tubular member 3 and the expansible member 2 are mutually fixed or connected with each other.

In the present invention, the means for fixing or connecting tubular member 3 and the expansible member 2 is not particularly limited. Specific examples of the fixing or connecting means may include direct suture or "hanging" using a polymer (e.g., synthetic polymer) material such as polypropylene mono filament fibers or polyester multi-filament fibers; or entanglement using fine metal fibers, etc. In view of adhesion property, it is preferred to connect or fix by the direct suture using the polyester multi-filament fibers.

Hereinbelow, the present invention will be described in more detail with reference to Examples.

### Examples

### Example 1

### (Manufacture of various artificial lumens)

An example of the stent-type artificial lumen (vascular prosthesis) according to the present invention is shown in Fig. 1. As an actual example thereof (trial product), a tube of a cloth having bellows was produced by using polyester fibers of ordinary diameter of 1.2 denier (50 mass %) and super ultra-fine polyester fibers of 0.2 denier (50 mass %). The inside diameter of the resultant product was 30 mm, and the length thereof was 10 cm. Into the thus obtained cloth tube, a Z-type stent for trachea formation which is expansible to a diameter of 40 mm was inserted as an example of stents, and the stent was sutured and fixed with the cloth tube by using 2-0 polyester multi-filament fibers. The cloth had a water-permeability of about 150 ml•cm⁻²•min⁻¹.

In this trial product, as shown in the schematic perspective view of Fig. 1, the tubular member 3 made of the cloth wherein super ultra-fine fibers have been mixed has a crimp structure due to heat-treatment. In the inside of the tubular member 3, the Z-type metal stent as a expansible member 2 is inserted and fixed with the tubular member 3.

Further, as shown in a schematic sectional view showing Fig. 2, the tubular member 3 has a plain weave structure based on fibers 6 (warp) and fibers 5 (woof) of ordinary diameter. In the clearances between the fibers of this cloth, super ultra-fine fibers denoted by the numerals 7 (longitudinal cross section), and 8 (lateral cross section; denoted by dots, because the cross section is extremely small) are mixed in the cloth. Super ultra-fine fibers 4a, 4b are raised to stand in the surface portion of the cloth. A portion of the raised super ultra-fine fibers have a loop-type structure 4b, and another portion is raised so as to expose their cut edges 4a. When the cloth is pressed, these raising fibers are easily flattened so that they do not increase the thickness of the whole cloth extremely.

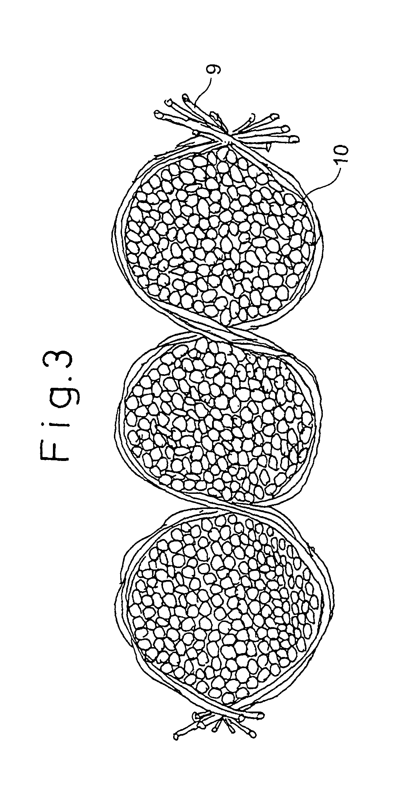

On the other hand, as a reference having a water-permeability near to the above trial product, there was provided an vascular prosthesis made of a cloth having the same diameter and the same length (mfd. by Ube Kosan Co., UBE-graft), and a Z-type stent was inserted into the inside of this vascular prosthesis and fixed therewith in the same manner as described above. The cloth showed a water-permeability of about 120 ml•cm⁻²•min⁻¹ (Reference product A). In this Reference product A, as shown in the schematic sectional view of Fig. 3, fibers of ordinary diameter (diameter, about 20 microns) are observed in a plain weave state. In this Reference product A, woof 9 and warp 10 are regularly crossed and densely interwoven, and there are very little clearances therebetween.

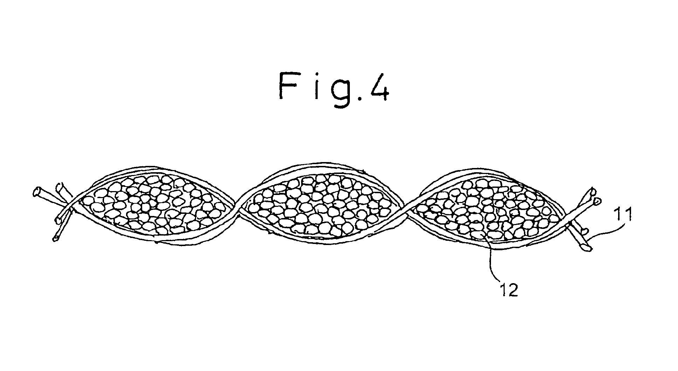

Separately, an vascular prosthesis made of a cloth (produced as trial products) which were liable to be inserted into a sheath (mfd. by Ube Kosan Co.) was provided, and another Reference product B having the same diameter and the same length was produced by using the vascular prosthesis. The cloth showed a water-permeability of about 250 ml•cm⁻²•min⁻¹ (Reference product B). In this Reference product B, as shown in the schematic sectional view of Fig. 4, fibers of ordinary diameter (diameter, about 20 microns) are observed in a plain weave state. In this Reference product A, woof 11 and warp 12 are regularly crossed and densely interwoven, and there are very little clearances therebetween. However, because the thickness of the entire cloth is relatively small, and water can leak from the intersection points of the woof and the warp.

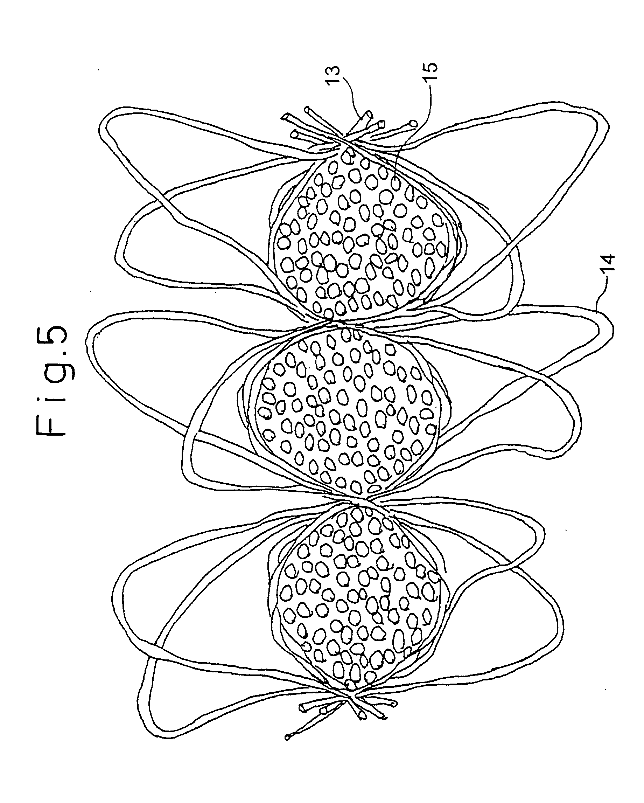

As a further Reference product, there was provided a commercially available vascular prosthesis having a raising structure. This only comprises fibers of ordinary diameter, and fibers of ordinary diameter provide the raising structure. Another Reference product C having same diameter and the same length was produced by using this cloth. The cloth showed a water-permeability of about 1200 ml•cm⁻²•min⁻¹ (Reference product C). In this Reference product C, as shown in the schematic perspective view of Fig. 5, fibers of ordinary diameter (diameter, about 20 microns) are observed in a plain weave state. In this Reference product C, woof 13 and warp 16 are regularly crossed, but the respective fibers are not interwoven densely, and a certain degree of fiber clearances are observed. In the portions of the warp 16, such a tendency is noticeable. A portion of the woof 13 is remarkably raised and forms a large loop 15 from the cloth. This is a cross section of the general cloth having a raising structure using fibers of ordinary diameter.

One end of each of the trial product and Reference product A was closed, and a vinyl chloride pipe was inserted into the other end of the product and the end was bound. Thereafter, 50ml of fresh blood taken out from a dog was injected into the inside space of each product. As a result, both of the vascular prosthesis were colored red, and it was seemed that blood can possibly leak out from these vascular prosthesis. However, the blood did not leak from the vascular prosthesis wall even when the blood is pressurized to 120 mmHg. Accordingly, it was found that the water-permeability's of both vascular prosthesis were 150 ml•cm⁻²•min⁻¹ and 120 ml•cm⁻²•min⁻¹, respectively, but both of them could prevent the leak of blood, and there was only a small difference between the water-permeabilities in view of practical use.

The test was conducted with respect to Reference product B in the same manner as described above. The blood continues leaking out from the vascular prosthesis wall, and the leak did not stop until the injection of the 50ml of blood was finished.

The test was conducted with respect Reference product C in the same manner as described above. The blood continues leaking out from the vascular prosthesis wall, and the leak did not stop until the injection of the 50ml of blood was finished, in the same manner as in the case of Reference product B.

### Example 2

With respect to each of the various artificial lumens produced in Example 1, the cantilever bending resistance Sa (mm), weight Sb (mg/cm²), water-permeability Sc (ml•cm⁻²•min⁻¹) and fraying-out resistance Sd (kg) were measured. Based on the thus measured values, the values of these products Q1 = (Sa × Sb × Sc) and the values of these combination Q2 = (Sa × Sb ÷ Sd) were calculated. The thus obtained results are shown in the following.

| <Artificial lumen> | <Trial product> | <Reference product> | <B> | <C> |
|---|---|---|---|---|
| Sa | 18 | 36 | 22 | 28 |
| Sb | 15.2 | 16.5 | 14.7 | 20.4 |
| Sc | 150 | 120 | 250 | 1200 |
| Sd | 1.5 | 0.5 | 0.2 | 1.0 |
| Q₁ | 41040 | 71280 | 80850 | 685440 |
| Q₂ | 182 | 1188 | 1617 | 571 |

### Example 3

### (In vitro insertion test)

In the clinical practices, a stent-type vascular prosthesis is inserted into a sheath, and is inserted into a blood vessel as it is. Then, the stent-type vascular prosthesis and sheath are moved forward to an intended site in the blood vessel by using a pusher, and the stent-type vascular prosthesis is first discharged into the blood vessel and placed therein. Accordingly, such a test was conducted in vitro. At first, the trial product and Reference product A were provided, and each of them was inserted in a sheath of 24 French having a length of 60 cm (mfd. by Cook company).

The cloth of the trial product was flexible and the folding thereof was easy, and it could be folded into a small and thin piece. Further, the insertion of the trial product into the sheath was also easy. On the other hand, the folding of Reference product A was difficult, and it was also difficult to fold it into a small piece. Finally, Reference product A was barely inserted into the sheath, by causing the cut edge of Reference product A into a funnel-like shape.

It was easy to move the trial product in a sheath, and the trial product was passed through the sheath of 60 cm without resistance by using a pusher. There was certain resistance in the insertion of Reference product A into the sheath, but the Reference product A could be passed through the sheath of 60 cm by forcedly pushing the pusher.

When these cloths which had passed through the sheath were observed with naked eyes, no noticeable change was observed. However, when a portion of both cloths was sampled and observed with a scanning electron microscope (magnification: 40 times, 100 times, 400 times, 1000 times, 3000 times), it was found that super ultra-fine polyester fibers of about 3 microns are raised from the surface of the trial product, and no disorder nor damage in the fibers was observed.

On the other hand, in the case of Reference product A, no noticeable change was recognized by the observation at a low magnification of 500 times or less, but when it was observed at a magnification of 1000 times or more, it was found that the diameter of polyester fibers was about 20 microns. When details of these fibers were observed, it was found that there were many fibers, a portion of which was flattened and damaged. However, no cutting of fibers was recognized.

By use of Reference product B, the passage thereof in a sheath was examined in the same manner as described above. The folding of Reference product B into a small and thin piece was easy, as in the case of the trial product, and the insertion of Reference product B into the sheath, the passage thereof in the sheath were also easy.

However, when the surface of Reference product B which had passed through the sheath was observed with naked eyes, with respect to a portion thereof which was just outside of the cut edge of the sheath, it was found that a plurality of metal portions of Z-type stent were penetrated into the cloth so as to be exposed from the outside of the cloth.

With respect to these portions, the metal portions returned to the inner space of the vascular prosthesis, when Reference product B was passed through the sheath and the stent was expanded. However, about 2 mm openings were left on the cloth at the penetration sites due to the metal.

A portion of Reference product B after the sheath passage test was taken out, and was observed with a scanning electron microscope (magnification: 1000 times). It was found that the diameter of the fiber was 20 microns, and the fibers were not damaged. However, it was also found that disorders in the knitting pattern were recognized everywhere, and severe disorders in the knitting pattern were recognized and the fibers were interspersed so as to provide large holes (even if no breakage of fibers was recognized), at the sites through which the metal had assumably been penetrated.

As describe above, the handling of the trial product was easy, the folding thereof into a small piece was easy, and it was possible that the trial product was passed through a sheath without receiving damage. On the contrary, Reference product A could not be inserted into a sheath unless it was forcedly inserted thereinto, and it was found that the polyester fibers were damaged by causing Reference product A to passe through the sheath.

On the other hand, the handling of Reference product B was easy, as in the case of the trial product. However, the knitting pattern of fibers was sparse, the fibers were easily scattered, and disorder was caused in the fiber structure with the metal of the stent so that openings were liable to be provided when the fibers receive stimuli based on the dull portion of the metal.

Further, the insertion of Reference product C into a sheath was attempted, but it was impossible to fold it into a small piece, and it was also completely impossible to insert Reference product C into the sheath.

### Example 4

### (Physical properties of various materials)

With respect to the following five kinds of ready-made cloths of fibers, various physical properties were measured. The results are shown in the following table.

The products used in this Example: Ube-graft (mfd. by Ube Kosan Co.), Toray graft (mfd. by Toray Industries, Inc.), Micron (mfd. by Intervascular Co.), Microknit (mfd. by Goraski Co.), Toray See (mfd. by Toray Industries, Inc.)

**Table 1**

| | Cantilever | Porosity | Catheter into which the cloth can be inserted (French) | Cloth thickness | Cloth weight | Suture Ret. | Degree of deformation | Q1 | Q2 |
|---|---|---|---|---|---|---|---|---|---|
| | mm | ml | | mm | mg/cm² | kg | | | |
| UBE-graft | 32 | 140 | 14 | 0.2 | 16.54 | 1.178 | easy | 74100 | 449 |
| Toray graft | 18 | 120 | 12 | 0.5 | 18.83 | 3.074 | difficult | 40680 | 110 |
| Micron | 28 | 1200 | 15 | 0.4 | 20.37 | 1.943 | easy | 796900 | 342 |
| Microknit | 18 | 4000 | 10 | 0.3 | 12.88 | 1.180 | easy | 928000 | 197 |
| Toray See | 23 | 160 | 9 | 0.2 | 7.74 | 1.387 | easy | 28040 | 129 |

### Example 5

By use of a simple plain weave cloth (mfd. by Ube Kosan Co.) which has actually been reported as a trial product, the product was thinned so as to provide a thickness of 0.2 mm, and the raising portion thereof was also suppressed so as to provide a product (tubular member) having a flat and smooth surface structure and an inside diameter of 20 mm. In the tubular member, a 24 mm-type Z-type stent (mfd. by Cook Co., trade name: Cook-Z (registered trademark) Stent; made of stainless steel, diameter of linear member : 0.5 mm) was inserted so as to provide a stent-type artificial lumen (vascular prosthesis) in a laboratory.

The thus obtained stent-type vascular prosthesis was slowly inserted into a sheath of 24 French (i.e., a diameter almost equivalent to the femoral artery of adult male; 50 cm length, material: Teflon; mfd. by Cook Co.; available from Medicos Hirata Co., or Tonokura Co.) for about 5 minutes, so that the entirety of the blood vessel was inserted into the sheath. After 60 minutes, the stent-type vascular prosthesis was taken out from the sheath. When the vascular prosthesis which had been taken out was observed, although an operation of cutting fibers was not conducted, it was found that a plurality of metal portions of the stent were penetrated into the cloth, and a plurality of clearances were formed in the tubular member due to the deformation in the fiber running in the cloth.

In the clinical practice, it is impossible to actually observe such a phenomenon, and further, it is impossible to prevent the phenomenon when a thin cloth which is liable to deform is used. Therefore, if the occurring clearances are large, they can cause bleeding, i.e., endoleak. This phenomenon is attributable to a fact that the cloth is thinned, so as to provide a simplified structure of fibers, whereby the density in the knitting and weaving of the fibers is deteriorated.

In the above, some Examples to which the present invention has been applied are described. However, the present invention should not be limited to these Examples, but various changes or modifications are possible in view of the shape or form, material, etc., unless such changes or modifications deviate from the features of the present invention.

### Industrial product Applicability

As described hereinabove, according to the present invention, there is provided an artificial lumen comprising a expansible member, and a tubular member of a porous material surrounding the expansible member; the artificial lumen having a product of Q1 = (Sa × Sb × Sc) of 6 × 10⁴ or less, provided that Sa (mm) denotes the bending resistance of the porous material by the cantilever bending resistance testing method, Sb (mg/cm²) denotes the weight of the porous material, and Sc (ml•cm⁻²•min⁻¹) denotes the water-permeability of the porous material according to the ANSI/AAMI standard.

The present invention also provides an artificial lumen comprising a expansible member, and a tubular member of a porous material surrounding the expansible member; the artificial lumen having a product of combination Q2 = (Sa × Sb ÷ Sd) of 300 or less, provided that Sa (mm) denotes the bending resistance of the porous material by the cantilever bending resistance testing method, Sb (mg/cm²) denotes the weight of the porous material, and Sd (kg) denotes the fraying-out resistance of the porous material by the suture retention test according to the ANSI/AAMI standard.

The present invention further provides an artificial lumen comprising a expansible member, and a tubular member of a porous material surrounding the expansible member; wherein the cloth comprises fibers of 0.8 denier or less in an amount of 1% or more by the weight ratio.

The stent-type artificial lumen according to the present invention having the above constitution uses a cloth satisfying a specific relationship between physical properties, instead of merely decreasing the thickness of the tubular member constituting a stent-type artificial lumen in the prior art. Accordingly, the present invention provides a stent-type artificial lumen wherein easy insertion thereof into the lumen of a living body, and exhibition of good characteristics as an artificial lumen after the insertion thereof into the lumen of a living body, are compatible with each other. That is, the present invention provides a stent-type artificial lumen wherein the tubular member thereof is thin but durable, and can easily maintain its original shape and form substantially; the artificial lumen can be folded into a small piece and can be inserted into a small-diameter sheath; and the artificial lumen can satisfy an affinity to the tissue of the living body, can retain thrombi residing in the wall thereof, and can satisfy an affinity to the cell of the living body, etc.

Because the stent-type artificial lumen according to the present invention comprises a thin and durable cloth, the insertion thereof into a sheath to be used for an ordinary operation is easy. Further, it is also possible to use a thinner sheath, and therefore the possibility of damaging the blood vessel wall is decreased and the unpleasant feeling of a patient due to the operation is reduced, whereby the operation can be conducted safely.

Because the stent-type artificial lumen according to the present invention comprises a thin and durable cloth, it is possible to prevent the damage to the cloth constituting the artificial lumen at the time of the passage thereof through the sheath, and to provide safe results of an operation in view of the long-term use thereof.

Further, in an embodiment wherein super ultra-fine fibers are raised, the stent-type artificial lumen according to the present invention is liable to capture thrombi on the wall of the artificial lumen, and can suppress the leak of blood from the artificial lumen wall.

In addition, in such an embodiment, the stent-type artificial lumen according to the present invention can induce the surrounding tissue in the living body into the raised super ultra-fine fibers, after the implantation thereof into the interior of the living body. As a result, the anchoring between the artificial lumen and the surrounding tissue can be ensured, the risk the graft migration can be suppressed to an extremely low level, and can realize safe results of an operation in a long-term point of view.

Further, in such an embodiment, it is expected that the stent-type artificial lumen according to the present invention can stably capture thrombi in the inside of the cloth, and can promote the integration or unification thereof with the connective tissue on the outside of the cloth. As a result, it is possible to prevent the leak of blood through the blood vessel wall, so-called endoleak, to realize a state of safe after the operation.

## Claims

1. An artificial lumen comprising a expansible member, and a tubular member of a porous material surrounding the expansible member;
the artificial lumen having a product Q1 = (Sa × Sb × Sc) of 6 × 10⁴ or less, provided that Sa (mm) denotes the bending resistance of the porous material by the cantilever bending resistance testing method, Sb (mg/cm²) denotes the weight of the porous material, and Sc (ml•cm⁻²•min⁻¹) denotes the water-permeability of the porous material according to the ANSI/AAMI standard.

2. An artificial lumen comprising a expansible member, and a tubular member of a porous material surrounding the expansible member;
the artificial lumen having a product of combination Q2 = (Sa × Sb ÷ Sd) of 300 or less, provided that Sa (mm) denotes the bending resistance of the porous material by the cantilever bending resistance testing method, Sb (mg/cm²) denotes the weight of the porous material, and Sd (kg) denotes the fraying-out resistance of the porous material by the suture retention test according to the ANSI/AAMI standard.

3. An artificial lumen comprising a expansible member, and a tubular member of a porous material surrounding the expansible member;
wherein the cloth comprises fibers of 0.8 denier or less in an amount of 1% or more by weight ratio.

4. An artificial lumen according to claim 1 or 2,
wherein the porous material is a cloth.

5. An artificial lumen according to claim 3 or 4,
wherein the cloth comprises fibers of 0.8 denier or less.

6. An artificial lumen according to any of claims 3 - 5, wherein at least a portion of the cloth has a raising structure.

7. An artificial lumen according to any of claims 3 - 6, wherein at least a portion of the cloth has a strength of 6g/d or more.

8. An artificial lumen according to any of claims 3 - 7, wherein the cloth has a pressure resistance of 20 kg or more by the burst test according to ANSI/AAMI standard.

9. An artificial lumen according to any of claims 3 - 8, wherein the cloth comprises fibers of a polymer selected from polyester, polyamide, polyolefin, or polytetrafluoroethylene.

10. An artificial lumen according to any of claims 3 - 9, wherein at least a portion of the fibers constituting the cloth have a randomly entangled state.

11. An artificial lumen according to any of claims 3 - 10, wherein the cloth has a knitted structure, a woven structure, a non-woven fabric structure, or a mixture of two or more kinds of these structures.

12. An artificial lumen according to any of claims 3 - 11, wherein the cloth has been subjected to a crimping treatment.

13. An artificial lumen according to any of claims 3 - 12, wherein the cloth has a water permeability of 2000 ml•cm⁻²•min⁻¹ or less.

14. An artificial lumen according to any of claims 3 - 13, wherein the cloth has an inside diameter of not less than 3 mm, and not more than 40 mm.

15. An artificial lumen according to any of claims 1 - 14, wherein at least a portion of the expansible member comprises a shape-memory alloy.

16. An artificial lumen according to any of claims 1 - 15, wherein at least a portion of the expansible member comprises a super elastic metal.

17. An artificial lumen according to any of claims 1 - 16, wherein the expansible member has a multi-filament structure.

18. An artificial lumen according to any of claims 1 - 17, wherein the expansible member has a belt-type structure.

19. An artificial lumen according to any of claims 1 - 18, wherein the expansible member comprises a synthetic polymer material.

20. An artificial lumen according to any of claims 1 - 19, wherein the expansible member has a self-expansion function.

21. An artificial lumen according to any of claims 1 - 20, wherein the expansible member has a function such that it can be expanded by a balloon.
